# EUROPÄISCHE PATENTSCHRIFT

(11) **EP 3 342 378 B1**
(45) Veröffentlichungstag und Bekanntmachung des Hinweises auf die Patenterteilung: **25.08.2021**
(21) Anmeldenummer: 17210774.0
(22) Anmeldetag: 28.12.2017
(51) Int. Cl.: A61F 7/02

(54) **MANSCHETTE ZUR VERWENDUNG BEI DER WÄRMETHERAPEUTISCHEN BEHANDLUNG DES MENSCHLICHEN KÖRPERS**
COLLAR FOR USE IN THE HEAT TREATMENT OF THE HUMAN BODY
MANCHETTE DESTINÉE À ÊTRE UTILISÉE DANS LE TRAITEMENT PAR LA CHALEUR DU CORPS HUMAIN

(30) Priorität: 03.01.2017 DE 102017200034
(43) Veröffentlichungstag der Anmeldung: 04.07.2018
(73) Patentinhaber: Hilotherm Holding AG, 6317 Oberwil bei Zug (CH)
(72) Erfinder: Janisch, Klaus, 88316 Isny (DE)
(74) Vertreter: Wallinger, Michael

(56) Entgegenhaltungen:
- EP-A1- 3 069 699
- US-A1- 2008 058 911
- US-A1- 2012 130 457
- US-A1- 2014 222 121
- US-A1- 2015 250 644

## Beschreibung

Die vorliegende Erfindung betrifft eine Manschette zur Verwendung bei thermotherapeutischen Behandlungen und ein System mit einer solchen Manschette.

Die thermotherapeutische Behandlung ist ein schon sehr lange bekanntes medizinisches Heilverfahren, bei dem dem Körper Wärme zugeführt wird - man spricht dann von Wärmetherapie - oder dem Körper Wärme entzogen wird - in diesem Fall spricht man von Kältetherapie. Um bei der Wärmetherapie Wärme auf den Körper übertragen zu können, ist die Wärmeübertragung von einer Wärmequelle auf den Körper erforderlich. Bei der Kältetherapie muss hingegen die Wärme dem Körper entzogen werden, d. h. es muss Wärme des Körpers auf eine Wärmesenke übertragen werden.

Die Wärmeübertragung kann in beiden Fällen direkt erfolgen, d. h. indem der Körper selbst in Kontakt mit einem wärmeübertragenden Medium gebracht wird, z. B. in einer Sauna oder in einem kalten Wasserbad. Daneben ist eine indirekte Wärmeübertragung möglich, bei welcher der Körper nicht selbst in Kontakt mit einem wärmeübertragenden Medium kommt. Die vorliegende Erfindung befasst sich mit der letztgenannten Vorgehensweise.

Bei der indirekten Wärmeübertragung wird ein Wärmeübertragungsmedium verwendet, das je nach Anwendung gasförmig oder flüssig, aber auch fest sein kann. Besonders bevorzugt ist als Wärmeübertragungsmedium Wasser, insbesondere destilliertes Wasser. Daneben können aber auch ein Gas oder eine Gasmischung und hier insbesondere Luft oder auch ein Öl oder ein Ölgemisch oder andere geeignete Flüssigkeiten oder Flüssigkeitsgemische zur Anwendung kommen.

Der Prozess der Wärmeübertragung kann diskontinuierlich oder kontinuierlich erfolgen. Eine bekannte diskontinuierliche Wärmeübertragungsanwendung ist der sogenannte Eisbeutel. Dazu wird Wasser zu Eis gefroren und in eine geeignete flexible, meist wasserdicht verschließbare Hülle eingefüllt. Der Eisbeutel wird auf den jeweiligen Körperteil aufgelegt und entzieht dem Körper so lange Wärme, bis das Eis aufgetaut ist und sich die Temperatur des Eisbeutels der Körpertemperatur angenähert hat.

Beim kontinuierlichen Verfahren wird das wärmeübertragende Medium mit einer Temperatur, die je nach Anwendung oberhalb oder unterhalb der Körpertemperatur liegt, einem Wärmeübertragungskörper zugeführt, der in Kontakt mit dem betroffenen Körperteil steht.

US 2012/0130457 A1 offenbart thermotherapeutische Pads, um eine temperaturkontrollierte Behandlung eines menschlichen Körperteils zu ermöglichen. Das Pad weist eine innere und eine äußere Oberfläche auf, welche einen gewundenen Kanal für ein Fluid zum Durchströmen des Pads aufweist. Temperaturkontrolliertes Fluid fließt dabei von einer Strömungseinrichtung über einen Einlass in das Pad, sodass das Fluid durch das gesamte Pad strömt, bevor es über einen Auslass zu der Strömungseinrichtung zurückströmt.

Der vorliegenden Erfindung liegt die Aufgabe zugrunde, eine Manschette mit wenigstens einem Wärmeübertragungskörper und ein System mit einer solchen Manschette zu schaffen, wobei der Wärmeübertragungskörper, je nach Gestaltung, für die kontinuierliche, die diskontinuierliche oder aber für beide Therapieformen geeignet ist.

Diese Aufgabe wird erfindungsgemäß durch den Gegenstand des Anspruchs 1 gelöst. Zu bevorzugende Weiterbildungen der Erfindung sind Gegenstand der Unteransprüche.

Das erfindungsgemäße System ist Gegenstand des Anspruchs 12.

Die Erfindung sieht eine Manschette zur Verwendung bei der wärmetherapeutischen Behandlung eines menschlichen Fußes vor, insbesondere zur Verwendung bei der Behandlung von Gewebeschädigungen nach operativen Eingriffen, von Verletzungen, von Entzündungen und von chronischen Erkrankungen des rheumatischen Formenkreises, und insbesondere bei der Chemotherapie mit zytostatischen Arzneistoffen bei der palmar-plantaren Erythrodysästhesie (PPE), wie z.B. Carboplatin, Capecitabin, 5-Fu, Cyclophosphamid, Cytarabin, Docetaxel, Doxorubicin, Oxaliplatin, Paclitaxel, Sorafinid und Sunitinib, mit einem Wärmeübertragungskörper, welcher in eine im Wesentlichen ebene Ausgangsform bringbar ist und wenigstens eine erste flexible Materiallage und wenigstens eine zweite flexible Materiallage aufweist, welche einen oder mehrere von einem wärmeübertragenden Fluid durchströmbare Strömungsräume begrenzen und durch eine Vielzahl von Kontaktierungseinrichtungen miteinander verbunden sind, und mit wenigstens einer ersten Zuleitung, um dem Wärmeübertragungskörper ein wärmeübertragendes Fluid zuzuführen, und wenigstens einer zweiten Zuleitung, um ein wärmeübertragendes Fluid von dem Wärmeübertragungskörper abzuführen.

Dabei ist der Wärmeübertragungskörper in der ebenen Ausgangsform in der Aufsicht asymmetrisch gestaltet und weist einen ersten Abschnitt, der im Wesentlichen den Umriss einer Fußsohle entspricht, und einen damit vorzugsweise stoffschlüssigen verbundenen zweiten Abschnitt, der derart ausgebildet ist, dass er sich in der Gebrauchsstellung über den Vorderfußbereich eines Benutzers erstreckt, auf.

Vorzugsweise sind die Kontaktierungseinrichtungen auf Zug und/oder Druck beanspruchbar.

Des Weiteren ist eine Verbindungseinrichtung vorgesehen, durch welche dieser erste Abschnitt mit diesem zweiten Abschnitt in der Gebrauchsstellung am Fuß des Benutzers gehalten ist. Auf diese Weise ist sichergestellt, dass die Manschette am Fuß des Benutzers flächig anliegt und damit eine gute Wärmeübertragung gewährleistet ist.

Unter einem Strömungsraum wird hierbei ein durchgehender Raum verstanden, in dem das wärmeübertragende Fluid für je zwei beliebige Punkte im Inneren des Raumes von einem der Punkte zu dem anderen Punkt strömen kann.

Die Manschette kann zum einmaligen oder auch zum mehrmaligen Gebrauch vorgesehen sein.

In einer bevorzugten Ausführung der Erfindung weist der Wärmeübertragungskörper eine der folgenden Formen auf oder kann eine dieser Formen annehmen:
- die Form einer den Fuß umschließenden Hülle ohne Unterteilungen des Innenraums;
- die Form eines Schuhes oder Strumpfes, welcher lediglich den Vorderfuß und die Fußsohle umschließt und/oder bedeckt.

Die Kontaktierungseinrichtungen, welche die erste und die zweite flexible Materiallage verbinden, sind insbesondere in kleinen und regelmäßigen Abständen an der Innenfläche der ersten flexiblen Materiallage und der Innenfläche der zweiten flexiblen Materiallage verteilt und dienen insbesondere dazu, ein Verschließen der Strömungsräume durch ein flächiges Aufeinanderliegen der ersten flexiblen Materiallage und der zweiten flexiblen Materiallage zu verhindern. Die Kontaktierungseinrichtungen sind vorzugsweise als Schweißpunkte ausgeführt, mit denen die erste flexible Materiallage mit der zweiten flexiblen Materiallage verschweißt und dadurch verbunden ist.

In einer bevorzugten Ausführung der Erfindung ist die Vielzahl von Kontaktierungseinrichtungen zumindest teilweise rasterförmig, vorzugsweise an den Eckpunkten von gedachten Rechtecken, weiter vorzugsweise an den Eckpunkten von gedachten Quadraten, angeordnet.

Ein solches Raster erleichtert das Design des Wärmeübertragungskörpers und sorgt dafür, dass ein Verschließen der Strömungsräume zwischen der ersten flexiblen Materiallage und der zweiten flexiblen Materiallage oder von Teilen davon gleichmäßig verhindert wird.

In einer bevorzugten Variante dieser Ausführung der Erfindung ist die wenigstens eine erste flexible Materiallage und die wenigstens eine zweite flexible Materiallage zwischen mindestens zwei Kontaktierungseinrichtungen, insbesondere zwischen den Mittelpunkten der beiden Kontaktierungseinrichtungen, linienförmig, insbesondere geradlinig, verbunden.

Derartige linienförmige Kontaktierungen lassen sich vorzugsweise auf besonders einfache und sichere Weise durch Verschweißen der ersten flexiblen Materiallage mit der zweiten flexiblen Materiallage als Schweißnähte herstellen und erfordern dann keine weiteren, separaten Elemente mehr zur Begrenzung der Strömungsräume.

Eine solche sich "überlagernde" Anordnung der linienförmigen Kontaktierungen zur Begrenzung der Strömungsräume und der Kontaktierungseinrichtungen ist vorteilhaft, da sonst Kontaktierungseinrichtungen sehr nah an den linienförmigen Kontaktierungen zu liegen kommen könnten und die durchströmbare Breite des Strömungsraumes an dieser Stelle unnötig stark eingeschränkt würde. Außerdem verhindern auch die linienförmigen Kontaktierungen selbst einen Verschluss der Strömungsräume, so dass eine unmittelbar benachbarte Anordnung von linienförmigen Kontaktierungen und Kontaktierungseinrichtungen unnötig ist.

Weiterhin bewirkt die "überlagernde" Anordnung von linienförmigen Kontaktierungen und Kontaktierungseinrichtungen - insbesondere dann, wenn letztere in einem Raster angeordnet sind - einen übersichtlichen und optisch ansprechenden Verlauf der Strömungsräume.

In einer bevorzugten Variante dieser Ausführung weist wenigstens eine der linienförmigen Kontaktierungen wenigstens einen geradlinigen Abschnitt auf. Vorzugsweise bestehen die linienförmigen Kontaktierungen zu mehr als 50 % ihrer Gesamtlänge, weiter vorzugsweise zu mehr als 90 % ihrer Gesamtlänge aus geradlinigen Abschnitten.

Dadurch sind die Werkzeuge zur Herstellung des Wärmeübertragungskörpers, insbesondere Werkzeugteile zum Pressen und Verschweißen der ersten flexiblen Materiallage und der zweiten flexiblen Materiallage miteinander entlang der linienförmigen Kontaktierungen, einfacher herzustellen. Auch in diesem Fall ergibt sich wieder ein übersichtlicher und optisch ansprechender Verlauf der Strömungsräume.

Erfindungsgemäß ist der Wärmeübertragungskörper in der ebenen Ausgangsform in der Aufsicht asymmetrisch gestaltet und weist einen ersten Abschnitt, der im Wesentlichen dem Umriss einer Fußsohle entspricht, und einen damit vorzugsweise stoffschlüssig verbundenen zweiten Abschnitt auf, der derart ausgebildet ist, dass er sich in der Gebrauchsstellung über den Vorderfußbereich eines Benutzers erstreckt.

Vorzugsweise ist der Wärmeübertragungskörper zusammenklappbar, wodurch er leichter herstellbar ist, und hat insbesondere die Form einer ebenen Fläche, in welcher die erste flexible Materiallage und die zweite flexible Materiallage miteinander verbunden werden. Weiterhin sind insbesondere die beiden flexiblen Materiallagen in der ebenen Ausgangsform des Wärmeübertragungskörpers leichter zu reinigen und/oder zu desinfizieren als in der Gebrauchsstellung.

In einer bevorzugten Variante dieser Ausführung weist der Wärmeübertragungskörper einen faltenartigen Übergangsbereich auf. Unter einem faltenartigen Übergangsbereich wird hierbei ein Bereich des Wärmeübertragungskörpers verstanden, in dem die erste flexible Materiallage und die zweite flexible Materiallage in der Gebrauchsstellung des Wärmeübertragungskörpers im Querschnitt eine Biegung mit einem kleinen Radius aufweisen.

Dass sich ein Strömungsraum über den faltenartigen Übergangsbereich hinweg erstreckt, hat den Vorteil, dass sich der gesamte Wärmeübertragungskörper dann mit nur einem durchgehenden Strömungsraum realisieren lässt und somit auch nur eine erste Zuleitung und eine zweite Zuleitung, welche auch keine Verzweigungen zu verschiedenen Strömungsräumen aufweisen müssen, nötig sind.

Bevorzugt erstrecken sich mehrere Strömungsräume oder mehrere Abschnitte desselben Strömungsraumes über den faltenartigen Übergangsbereich hinweg.

Bevorzugt sind mehrere sich über den faltenartigen Übergangsbereich hinweg erstreckende Abschnitte desselben Strömungsraumes auf einer oder auf beiden Seiten des faltenartigen Übergangsbereichs durch je einen Abschnitt dieses Strömungsraumes verbunden, welcher jeweils im Wesentlichen entlang des faltenartigen Übergangsbereichs verläuft.

Bevorzugt ist wenigstens eine Kontaktierungseinrichtung auf dem faltenartigen Übergangsbereich innerhalb eines Strömungsraumes, welche sich über den faltenartigen Übergangsbereich hinweg erstreckt, angeordnet.

In einer weiteren bevorzugten Ausführung ist der Strömungsraum an wenigstens einem Punkt einer linienförmigen Kontaktierung, insbesondere im Bereich des faltenartigen Übergangsbereichs, unterbrochen, so dass das Fluid zwischen zwei Strömungsräumen fließen kann, um eine ununterbrochene Strömung des Fluids zu gewährleisten.

Erfindungsgemäß ist bei Strömungsräumen, insbesondere bei kritischen Strömungsräumen, bei welchen, insbesondere durch Knick- und/oder Drehbewegungen des menschlichen Fußes, ein Falz in dem Strömungsraum entsteht, das Fluid an mindestens einer unterbrochenen Stelle einer linienförmigen Kontaktierung zwischen zwei Strömungsräumen fließen kann, um eine ununterbrochene Strömung des Fluids zu gewährleisten.

In einer weiteren bevorzugten Ausführung ist wenigstens eine parallele Führung des Fluids in wenigstens zwei Strömungsräumen in einem vorbestimmten Winkel, insbesondere senkrecht oder parallel, zum Falz möglich, um eine ununterbrochene Strömung des Fluids zu gewährleisten.

Bevorzugt ist die entlang des faltenartigen Übergangsbereichs gemessene Gesamtbreite eines sich über den faltenartigen Übergangsbereich hinweg erstreckenden Strömungsraumes größer als, insbesondere wenigstens doppelt so groß wie, die senkrecht zur Strömungsrichtung gemessene Gesamtbreite dieses Strömungsraumes in einem in Strömungsrichtung gesehen unmittelbar vor oder nach dem faltenartigen Übergangsbereich angeordneten Abschnitt dieses Strömungsraumes.

Die genannten Maßnahmen dienen - einzeln oder in Kombination - dazu, die Querschnittsfläche des oder der sich über den faltenartigen Übergangsbereich hinweg erstreckenden Strömungsräume gezielt zu vergrößern, um einer Verringerung der Höhe der Strömungsräume, welche durch das Zusammenklappen des

Wärmeübertragungskörpers entlang des faltenartigen Übergangsbereichs verursacht werden kann, entgegenzuwirken. Durch eine solche Höhenverringerung der Strömungsräume könnte die Menge des wärmeübertragenden Fluids, welche pro Zeiteinheit durch die Strömungsräume strömen kann, und damit auch die Wärmeübertragung selbst verringert werden.

In einer weiteren bevorzugten Ausführung der Erfindung ist der zweite Abschnitt asymmetrisch im Vergleich zum ersten Abschnitt und insbesondere trapezförmig mit geraden und/oder stetig gekrümmten Seitenkanten ausgebildet.

Erfindungsgemäß ist eine Verbindungseinrichtung vorgesehen, durch die der erste Abschnitt mit dem zweiten Abschnitt so verbindbar oder so verbunden ist, dass die Manschette in der Gebrauchsstellung am Fuß des Benutzers gehalten ist.

In einer bevorzugten Ausführung der Erfindung ist die Verbindungseinrichtung lösbar.

Bevorzugt weist die Verbindungseinrichtung wenigstens ein Fixierungselement, insbesondere wenigstens einen Druckknopf und/oder einen Klettverschluss und/oder eine Schlaufe zum Binden und/oder ein elastisches Band, auf, um den ersten Abschnitt mit dem zweiten Abschnitt in der Gebrauchsstellung miteinander lösbar zu verbinden.

Dadurch kann der Wärmeübertragungskörper wiederholt geöffnet, gereinigt und/oder desinfiziert sowie wieder verschlossen werden, wodurch der Zeitraum der Verwendbarkeit der Manschette verlängert werden kann.

Es ist jedoch auch möglich, dass wenigstens zwei Abschnitte des Wärmeübertragungskörpers unlösbar, beispielsweise durch Verschweißen, miteinander verbunden sind. Dies erhöht die Stabilität der Manschette und die Wärmeisolation des zu behandelnden Körperteils gegenüber der Umgebung.

In einer bevorzugten Variante dieser Ausführung sind die wenigstens zwei Abschnitte des Wärmeübertragungskörpers auf verschiedene Weise miteinander lösbar verbindbar, so dass dabei verschiedene Größen des Wärmeübertragungskörpers entstehen, welche insbesondere verschiedenen Größen des zu behandelnden Körperteils entsprechen.

Vorzugsweise ist es möglich, dass die Verbindungseinrichtung mehrere Fixierungselemente, welche insbesondere versetzt zueinander angeordnet sind, aufweist, um eine variable Größenanpassung an den Fuß zu ermöglichen. Hierdurch ergibt sich eine Vereinfachung bei der Herstellung, der Lagerhaltung und dem Vertrieb der Manschette als kommerziellem Produkt, da das Produkt dann nur noch in wenigen oder sogar nur noch in einer Version hergestellt, vorgehalten und ausgeliefert werden muss, ohne dass die Körpermaße der Patienten, insbesondere deren verschiedene Fußgrößen, berücksichtigt werden müssen ("one size fits all").

Des Weiteren ist es vorzugsweise möglich, dass die Verbindungseinrichtung derart ausgebildet ist, dass sie die Manschette in der Gebrauchsstellung zumindest teilweise umgreift und in dieser umgreifenden Stellung Hilfskräfte auf die Manschette ausübt, welche zumindest teilweise dazu beitragen, die Manschette in der Gebrauchsstellung zu halten.

Vorzugsweise erfolgt die Verbindung der wenigstens zwei Abschnitte des Wärmeübertragungskörpers miteinander in der Gebrauchsstellung des Wärmeübertragungskörpers.

In einer weiteren bevorzugten Ausführung sind die erste Zuleitung und die zweite Zuleitung im Wesentlichen parallel zueinander an den Wärmeübertragungskörper angeschlossen. Vorteilhaft ist hierbei, dass die beiden Zuleitungen gemeinsam vom und zum Benutzer geführt werden können und eine einfache Handhabung erfolgen kann.

Ein erfindungsgemäßes Wärmebehandlungssystem zur Verwendung bei der wärmetherapeutischen Behandlung eines Teiles des menschlichen Körpers weist eine erfindungsgemäße Manschette sowie eine Fluidversorgungseinrichtung auf, welche mit der wenigstens einen ersten Zuleitung und der wenigstens einen zweiten Zuleitung der Manschette fluidleitend verbindbar ist und welche dazu eingerichtet ist, ein wärmeübertragendes Fluid auf eine für die Wärmebehandlung geeignete Temperatur, bevorzugt eine Temperatur zwischen +5 °C und +35 °C, weiter bevorzugt zwischen +10 °C und +20 °C, besonders bevorzugt zwischen +14 °C und +16 °C und ganz besonders bevorzugt um ca. +15 °C, zu temperieren, das derart temperierte wärmeübertragende Fluid dem Wärmeübertragungskörper der Manschette über die erste Zuleitung zuzuführen und das wärmeübertragende Fluid von dem Wärmeübertragungskörper über die zweite Zuleitung abzuführen.

Die Verwendung der Manschette und/oder des Wärmebehandlungssystems kann außerdem zur kosmetischen Behandlung der Haut des menschlichen Fußes benutzt werden. Hierzu zählen Anwendungen hinsichtlich der Faltenbehandlung bzw. der Faltenreduktion, der thermischen Beeinflussung der Kapillarzeichnung auf der Haut und/oder auch die nicht-therapeutische Reduktion von Hautschwellungen.

Darüber hinaus ist eine Verwendung der Manschette und/oder des Wärmebehandlungssystems zur thermisch bewirkten Reduzierung der Bildung freier Radikale auf der Hautoberfläche möglich.

Weitere vorteilhafte Ausgestaltungen der Erfindung werden in der nachfolgenden Beschreibung in Zusammenhang mit der beiliegenden Zeichnung erläutert. Dabei zeigt:

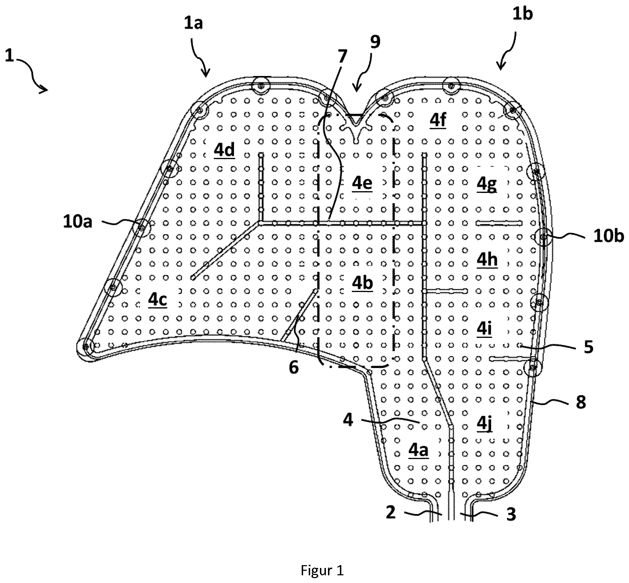

**Fig. 1****:** eine erfindungsgemäße Manschette in Schuhform in der Ausgangsform.

**Fig. 1** zeigt eine erfindungsgemäße Manschette mit einem Wärmeübertragungskörper 1 in der Ausgangsform, wodurch der erste Abschnitt 1a und der zweite Abschnitt 1b, welcher asymmetrisch im Vergleich zum ersten Abschnitt 1a angeordnet ist, des Wärmeübertragungskörpers 1 sichtbar sind. Weiterhin weist die Manschette eine erste Zuleitung 2 und eine zweite Zuleitung 3 auf, durch welche ein wärmeleitendes, temperiertes Fluid, insbesondere erwärmtes oder gekühltes Wasser, durch eine (nicht gezeigte) Fluidversorgungseinrichtung zu- bzw. abführbar ist.

Der Wärmeübertragungskörper 1 lässt sich entlang eines faltenartigen Übergangsbereichs 9 zusammenklappen und nimmt in der Gebrauchsstellung die Form eines Schuhs, insbesondere eines nur im Bereich des Vorderfußes geschlossenen Schuhs (d. h. einer "Pantoffel"), an.

Der Wärmeübertragungskörper 1 weist eine erste flexible Materiallage und eine zweite flexible Materiallage auf, welche deckungsgleich angeordnet sind und von denen in der Aufsicht in **Fig. 1** lediglich die Innenseite der ersten flexiblen Materiallage sichtbar ist. Die beiden Materiallagen sind vorzugsweise aus Kunststoff gefertigt. Im Ausführungsbeispiel gemäß **Fig. 1** sind die beiden Materiallagen weitgehend transparent.

An ihren äußeren Rändern sind die beiden Materiallagen durch eine Randschweißnaht 8, welche nahezu lückenlos um den gesamten Wärmeübertragungskörper 1 herum verläuft, fluiddicht zusammengeschweißt.

Die Randschweißnaht 8 wird lediglich an den Durchführungsstellen einer ersten Zuleitung 2 und einer zweiten Zuleitung 3 unterbrochen, wo sie stark verbreitert sein kann, um die Enden der ersten und der zweiten Zuleitung 2, 3 sicher zu halten und fluiddicht abzudichten. Die Durchführungsstellen der ersten und der zweiten Zuleitung 2, 3 sind im Bereich des in **Fig. 1** unteren Endes des Wärmeübertragungskörpers 1 an der offenen Seite des Schuhs (entsprechend der Ferse) angeordnet. Des Weiteren sind die Zuleitungen 2, 3 vorzugsweise parallel zueinander angeordnet.

Auf der Randschweißnaht 8 sind in bestimmten, teilweise äquidistanten Abständen Druckknopfhälften 10a, 10b angebracht, auf der ersten Hälfte 1a des Wärmeübertragungskörpers 1 die steckerartigen und in der zweiten Hälfte 1b die buchsenartigen Hälften oder umgekehrt. Nach dem Zusammenklappen des Wärmeübertragungskörpers 1 lässt sich jeweils eine Druckknopfhälfte 10a in eine Druckknopfhälfte 10b oder umgekehrt einrasten. Dadurch nimmt der Wärmeübertragungskörper 1 die Gebrauchsstellung und somit die Form eines Schuhs an, in welchen der Patient seinen Fuß stecken kann, um die Wärmetherapie anzuwenden.

Auch könnten die steckerartigen und/oder die buchsenartigen Druckknopfhälften 10a, 10b versetzt, insbesondere jeweils in mehreren Reihen, angeordnet sein, damit die Druckknopfhälften 10a, 10b auf verschiedene Weise ineinander einrasten können und sich dadurch für den Wärmeübertragungskörper 1 verschiedene Schuhgrößen ergeben.

Die erste und die zweite Materiallage sind auf ihrer gesamten Fläche, abgesehen von der Randschweißnaht 8, durch eine Vielzahl von Schweißpunkten 5 miteinander verbunden, welche in einem quadratischen Raster angeordnet sind. Der Abstand zwischen benachbarten Schweißpunkten in horizontaler oder vertikaler Richtung wird im Folgenden als "Rasterabstand r" bezeichnet.

Durch weitere Schweißnähte im Flächeninneren der ersten und zweiten Materiallage, durch welche die erste und die zweite Materiallage fluiddicht miteinander verbunden sind, ist eine Mehrzahl von Kanalwänden 6 gebildet. Die Kanalwände 6 bestehen zum größten Teil aus sich aneinander anschließenden, geradlinigen Abschnitten. Insbesondere verbinden die Kanalwände 6 jeweils eine Anzahl von benachbarten Schweißpunkten miteinander.

Durch die Kanalwände 6 wird in dem Wärmeübertragungskörper 1 zwischen der ersten und der zweiten Materiallage ein einziger, durchgehender, fluidleitender Kanal 4 gebildet.

Der Kanal 4 beginnt in der ersten Hälfte 1a des Wärmeübertragungskörpers 1 am Ende der ersten Zuleitung 2 und wird durch den Abschnitt 4a beschrieben, welcher eine Breite von ca. 5 raufweist. Dieser erste Abschnitt 4a, welcher den hinteren linken Teil der Fußsohle temperiert, führt das Fluid zu dem faltenartigen Übergangsbereich 9, bei dem der Abschnitt 4b in der Breite deutlich vergrößert wird. An der breitesten Stelle weist der Abschnitt 4b eine Breite von 16 *r* auf, gemessen quer zur Strömungsrichtung innerhalb des Kanals 4. Dies wirkt einer Verringerung der Höhe der Strömungsräume, welche durch das Zusammenklappen des Wärmeübertragungskörpers entlang des faltenartigen Übergangsbereichs verursacht werden kann, entgegen.

Des Weiteren kann das Fluid an einer lückenhaften Kanalwand 7 von Abschnitt 4b zu Abschnitt 4e strömen. Dies kann in dem faltenartigen Übergangsbereich 9 in der Gebrauchsstellung, bei dem der Fluidstrom möglicherweise stark reduziert oder unterbrochen ist, den Fluidstrom positiv beeinflussen, so dass eine dauerhafte Zirkulation des Fluids gewährleistet ist.

Durch diagonal angeordnete Kanalwände 6 wird das Fluid weiter über die beiden Abschnitte 4c und 4d geleitet, welche die Oberseite des Vorderfußes temperieren, und gelangt in Abschnitt 4e, welcher auch im Bereich des faltenartigen Übergangsbereichs liegt. Dieser Abschnitt ist wie Abschnitt 4b durch einen deutlich breiteren Strömungskanal 4 gekennzeichnet, welcher an der breitesten Stelle eine Breite von 12 r aufweist, gemessen quer zur Strömungsrichtung innerhalb des Kanals 4.

Sodann geht der Kanal 4 über den Abschnitt 4f, welcher unter den Zehen des Fußes angeordnet ist, in die Abschnitte 4g, 4h sowie 4i über. Diese Abschnitte, welche im Durchschnitt eine Breite von 8 *r* aufweisen, sind im Bereich der Fußsohle des Vorderfußes angeordnet. Ferner sind in den Abschnitten 4g, 4h sowie 4i Kanalwände 6 quer zum Fluidstrom ausgebildet, um den Fluidstrom in der Zirkulation zu beeinflussen. Diese Kanalwände 6 sind als lückenhafte Kanalwände 7 ausgebildet, um den Fluidstrom z. B. an eine bestimmte Fußsohlenform anpassen zu können. Insbesondere kann durch die lückenhaften Kanalwände 7 die Strömungsgeschwindigkeit des Fluids beeinflusst, insbesondere verringert, werden.

Daran schließt sich der Abschnitt 4j an, welcher im Bereich der Ferse des Benutzers angeordnet ist und das Fluid zur zweiten Zuleitung 3 führt, um das Fluid vom Wärmeübertragungskörper wieder abzuführen.

### Bezugszeichenliste

- 1: Wärmeübertragungskörper
- 1a: Erste Hälfte des Wärmeübertragungskörpers
- 1b: Zweite Hälfte des Wärmeübertragungskörpers
- 2: Erste Zuleitung
- 3: Zweite Zuleitung
- 4: fluidleitender Kanal
- 4a - 4j: Kanalabschnitte
- 5: Schweißpunkt / Kontaktierungseinrichtung
- 6: Kanalwand
- 7: lückenhafte Kanalwand
- 8: Randschweißnaht
- 9: Faltenartiger Übergangsbereich
- 10a: Steckerartige Druckknopfhälfte
- 10b: Buchsenartige Druckknopfhälfte

## Patentansprüche

1. Manschette zur Verwendung bei der wärmetherapeutischen Behandlung des menschlichen Fußes, insbesondere zur Verwendung bei der Behandlung von Gewebeschädigungen nach operativen Eingriffen, von Verletzungen, von Entzündungen und von chronischen Erkrankungen des rheumatischen Formenkreises, und insbesondere bei der Chemotherapie mit zytostatischen Arzneistoffen bei der palmar-plantaren Erythrodysästhesie (PPE), wie z.B. Carboplatin, Capecitabin, 5-Fu, Cyclophosphamid, Cytarabin, Docetaxel, Doxorubicin, Oxaliplatin, Paclitaxel, Sorafinid und Sunitinib, mit einem Wärmeübertragungskörper (1), welcher in eine im Wesentlichen ebene Ausgangsform bringbar ist und wenigstens eine erste flexible Materiallage und wenigstens eine zweite flexible Materiallage aufweist, welche einen oder mehrere von einem wärmeübertragenden Fluid durchströmbare Strömungsräume (4) begrenzen und durch eine Vielzahl von Kontaktierungseinrichtungen (5) miteinander verbunden sind, und mit wenigstens einer ersten Zuleitung (2), um dem Wärmeübertragungskörper (1) ein wärmeübertragendes Fluid zuzuführen, und mit wenigstens einer zweiten Zuleitung (3), um ein wärmeübertragendes Fluid von dem Wärmeübertragungskörper (1) abzuführen,
wobei der Wärmeübertragungskörper (1) in der ebenen Ausgangsform in der Aufsicht asymmetrisch gestaltet ist **dadurch gekennzeichnet, dass** der Wärmeübertragungskörper einen ersten Abschnitt aufweist, der im Wesentlichen dem Umriss einer Fußsohle entspricht, sowie einen damit vorzugsweise stoffschlüssig verbundenen zweiten Abschnitt, der derart ausgebildet ist, dass er sich in der Gebrauchsstellung über den Vorderfußbereich eines Benutzers erstreckt,
und dass weiterhin eine Verbindungseinrichtung vorgesehen ist, durch welche dieser erste Abschnitt mit diesem zweiten Abschnitt so verbindbar oder so verbunden ist, dass die Manschette in der Gebrauchsstellung am Fuß des Benutzers gehalten ist,
wobei bei Strömungsräumen (4), insbesondere bei kritischen Strömungsräumen (4), bei welchen, insbesondere durch Knick- und/oder Drehbewegungen des menschlichen Fußes, ein Falz in dem Strömungsraum (4) entsteht, das Fluid an mindestens einer unterbrochenen Stelle einer linienförmigen Kontaktierung (6) zwischen zwei Strömungsräumen (4) fließen kann, um eine ununterbrochene Strömung des Fluids zu gewährleisten.

2. Manschette nach Anspruch 1, **dadurch gekennzeichnet, dass** der zweite Abschnitt asymmetrisch im Vergleich zum ersten Abschnitt und insbesondere trapezförmig mit geraden und/oder stetig gekrümmten Seitenkanten ausgebildet ist.

3. Manschette nach wenigstens einem der vorhergehenden Ansprüche, **dadurch gekennzeichnet, dass** die Verbindungseinrichtung wenigstens ein Fixierungselement, insbesondere wenigstens einen Druckknopf und/oder einen Klettverschluss und/oder eine Schlaufe zum Binden und/oder ein elastisches Band und/oder eine Schweißnaht, aufweist.

4. Manschette nach Anspruch 3, **dadurch gekennzeichnet, dass** die Verbindungseinrichtung mehrere Fixierungselemente aufweist, welche insbesondere versetzt zueinander angeordnet sind, um eine variable Größenanpassung an den Fuß zu ermöglichen.

5. Manschette nach wenigstens einem der vorhergehenden Ansprüche, **dadurch gekennzeichnet, dass** die Verbindungseinrichtung derart ausgebildet ist, dass sie die Manschette in der Gebrauchsstellung zumindest teilweise umgreift und in dieser umgreifenden Stellung Hilfskräfte auf die Manschette ausübt, welche zumindest teilweise dazu beitragen, die Manschette in der Gebrauchsstellung zu halten.

6. Manschette nach wenigstens einem der vorhergehenden Ansprüche, **dadurch gekennzeichnet, dass** die Vielzahl von Kontaktierungseinrichtungen (5) zumindest teilweise rasterförmig, vorzugsweise an den Eckpunkten von gedachten Rechtecken, weiter vorzugsweise an den Eckpunkten von gedachten Quadraten, angeordnet ist.

7. Manschette nach wenigstens einem der vorhergehenden Ansprüche, **dadurch gekennzeichnet, dass** die wenigstens eine erste flexible Materiallage und die wenigstens eine zweite flexible Materiallage zwischen mindestens zwei Kontaktierungseinrichtungen (5), insbesondere zwischen den Mittelpunkten der beiden Kontaktierungseinrichtungen (5), linienförmig, insbesondere geradlinig, verbunden sind.

8. Manschette nach einem der vorhergehenden Ansprüche, **dadurch gekennzeichnet, dass** wenigstens eine parallele Führung des Fluids in wenigstens zwei Strömungsräumen in einem vorbestimmten Winkel, insbesondere senkrecht oder parallel, zum Falz ermöglicht ist, um eine ununterbrochene Strömung des Fluids zu gewährleisten.

9. Manschette nach einem der vorhergehenden Ansprüche, **dadurch gekennzeichnet, dass** mindestens ein Strömungsraum (4) in der Breite variiert ist, insbesondere vergrößert ist, um eine ununterbrochene Strömung des Fluids zu gewährleisten.

10. Manschette nach wenigstens einem der vorhergehenden Ansprüche, **dadurch gekennzeichnet, dass** die erste Zuleitung (2) und die zweite Zuleitung (3) im Wesentlichen parallel zueinander an den Wärmeübertragungskörper angeschlossen sind.

11. Wärmebehandlungssystem zur Verwendung bei der wärmetherapeutischen Behandlung eines menschlichen Fußes mit einer Manschette nach wenigstens einem der Ansprüche 1 bis 10 und einer Fluidversorgungseinrichtung, die zur fluidleitenden Verbindung mit der Manschette eingerichtet ist und welche mit der wenigstens einen ersten Zuleitung (2) und der wenigstens einen zweiten Zuleitung (3) der Manschette fluidleitend verbindbar ist und welche dazu eingerichtet ist, ein wärmeübertragendes Fluid auf eine für die Wärmebehandlung geeignete Temperatur, bevorzugt eine Temperatur zwischen +5 °C und +35 °C, weiter bevorzugt zwischen +10 °C und +20 °C, besonders bevorzugt zwischen +14 °C und +16 °C und ganz besonders bevorzugt um ca. +15 °C, zu temperieren, das derart temperierte wärmeübertragende Fluid dem Wärmeübertragungskörper (1) der Manschette über die erste Zuleitung (2) zuzuführen und das wärmeübertragende Fluid von dem Wärmeübertragungskörper (1) über die zweite Zuleitung (3) abzuführen.

12. Verwendung einer Manschette nach einem der Ansprüche 1 bis 10 und/oder eines Wärmebehandlungssystems nach Anspruch 11 zur kosmetischen Behandlung der Haut des menschlichen Fußes, insbesondere zur
- Faltenbehandlung/-reduktion oder
- zur thermischen Beeinflussung der Kapillarzeichnung auf der Haut oder
- zur nicht-therapeutischen Reduktion von Hautschwellungen.

## Claims

1. Sleeve for use in the heat-therapeutic treatment of the human foot, in particular for use in the treatment of tissue damage after surgical interventions, of injuries, of injuries, of inflammations and of chronic rheumatic diseases, and in particular in chemotherapy with cytostatic drugs in palmar-plantar erythrodysesthesia (PPE), e.g. carboplatin, capecitabine, 5-Fu, cyclophosphamide, cytarabine, docetaxel, doxorubicin, oxaliplatin, paclitaxel, sorafinide, and sunitinib, comprising a heat transfer body (1) which can be brought into a substantially planar initial shape and comprises at least one first flexible material layer and at least one second flexible material layer which confine one or more flow spaces (4) through which a heat-transmitting fluid can flow, and which are connected to one another by a plurality of contacting devices (5) and comprising at least one first supply line (2) for supplying a heat-transmitting fluid to the heat transfer body (1) and with at least one second supply line (3) for discharging a heat-transmitting fluid from the heat transfer body (1), wherein the heat transfer body (1) is asymmetrically shaped in plan view in the planar initial shape,
**characterized in that** the heat transfer body comprises a first section which substantially corresponds to the outline of a sole of a foot, and a second section which is connected thereto, preferably in a bonded manner, and which is designed such that, in the position of use, it extends over the forefoot region of a user, and **in that** furthermore a connecting device is provided, by means of which this first section is connectable or connected to this second section such that the sleeve is held on the foot of the user in the position of use,
wherein at flow spaces (4), in particular at critical flow spaces (4), in which, in particular as a result of buckling and/or rotational movements of the human foot, a fold in the flow space (4) is formed, the fluid can flow at at least one interrupted point of a linear contact (6) between two flow spaces (4) in order to ensure an uninterrupted flow of the fluid.

2. Sleeve according to claim 1, **characterized in that** the second section is formed asymmetrically compared to the first section and in particular trapezoidally with straight and/or continuously curved side edges.

3. Sleeve according to at least one of the preceding claims, **characterized in that** the connecting device comprises at least one fixing element, in particular at least one snap fastener and/or one Velcro fastener and/or one loop for tying and/or one elastic band and/or one welded seam.

4. Sleeve according to claim 3, **characterized in that** the connecting device comprises several fixing elements which are arranged in particular offset with respect to one another in order to allow variable size adaptation to the foot.

5. Sleeve according to at least one of the preceding claims, **characterized in that** the connecting device is designed such that it at least partially engages around the sleeve in the position of use and, in this engaging position, exerts auxiliary forces on the sleeve which at least partially contribute to holding the sleeve in the position of use.

6. Sleeve according to at least one of the preceding claims, **characterized in that** the plurality of contacting devices (5) is arranged at least partially in a grid-like manner, preferably at the corner points of imaginary rectangles, further preferably at the corner points of imaginary squares.

7. Sleeve according to at least one of the preceding claims, **characterized in that** the at least one first flexible material layer and the at least one second flexible material layer are connected in a linear, in particular rectilinear, manner between at least two contacting devices (5), in particular between the center points of the two contacting devices (5).

8. Sleeve according to one of the preceding claims, **characterized in that** at least one parallel guidance of the fluid in at least two flow spaces is facilitated at a predetermined angle, in particular perpendicular or parallel, to the fold, in order to ensure an uninterrupted flow of the fluid.

9. Sleeve according to one of the preceding claims, **characterized in that** at least one flow space (4) is varied in width, in particular enlarged, in order to ensure an uninterrupted flow of the fluid.

10. Sleeve according to at least one of the preceding claims, **characterized in that** the first supply line (2) and the second supply line (3) are connected to the heat transfer body substantially parallel to each other.

11. Heat treatment system for use in the heat-therapeutic treatment of a human foot, comprising a sleeve according to at least one of claims 1 to 10, and a fluid supply device which is adapted to be fluidly connected to the sleeve and which is fluidly connectable to the at least one first supply line (2) and the at least one second supply line (3) of the sleeve and which is adapted to maintain the temperature of a heat-transmitting fluid at a temperature suitable for the heat treatment, preferably a temperature between +5 °C and +35 °C, further preferably between +10 °C and +20 °C, more preferably between +14 °C and +16 °C and even more preferably around approximately +15 °C, to supply the heat-transmitting fluid whose temperature is thus maintained to the heat transfer body (1) of the sleeve via the first supply line (2) and to discharge the heat-transmitting fluid from the heat transfer body (1) via the second supply line (3).

12. Use of a sleeve according to any one of claims 1 to 10 and/or of a heat treatment system according to claim 11 for the cosmetic treatment of the skin of the human foot, in particular for
- the treatment/reduction of wrinkles or
- for thermally influencing the capillary drawing on the skin or
- for the non-therapeutic reduction of skin swellings.

## Revendications

1. Manchon destiné à être utilisé lors du traitement par thermothérapie du pied humain, en particulier destiné à être utilisé lors du traitement de lésions tissulaires après des interventions chirurgicales, de blessures, d'inflammations et d'affections chroniques du type rhumatismal, et en particulier lors de la chimiothérapie avec des médicaments cystostatiques dans le cas de l'érythrodysesthésie palmaire et plantaire (EPP), comme le carboplatine, la capécitabine, le 5-Fu, le cyclophosphamide, la cytarabine, le docétaxel, la doxorubicine, l'oxaliplatine, le paclitaxel, le sorafénib, le sunitinib, avec un corps de transfert de chaleur (1), lequel peut être amené sous une forme de départ sensiblement plane et présente au moins une première couche de matériau flexible et au moins une deuxième couche de matériau flexible, lesquelles délimitent un ou plusieurs espaces d'écoulement (4) pouvant être traversés par un fluide de transfert de chaleur et sont reliées l'une à l'autre par une pluralité de dispositifs de mise en contact (5), et avec au moins un premier conduit d'arrivée (2) pour amener au corps de transfert de chaleur (1) un fluide de transfert de chaleur, et avec au moins un deuxième conduit d'arrivée (3) pour évacuer un fluide de transfert de chaleur du corps de transfert de chaleur (1),
dans lequel le corps de transfert de chaleur (1) est configuré dans la forme de départ plane de manière asymétrique vu d'en haut, **caractérisé en ce que** le corps de transfert de chaleur présente une première section, qui correspond sensiblement au contour d'une plante de pied, ainsi qu'une deuxième section reliée à la première de préférence par liaison de matière, qui est réalisée de telle manière qu'elle s'étend, dans la position d'utilisation, au-delà de la zone de l'avant-pied d'un utilisateur,
et qu'est prévu par ailleurs un dispositif de liaison, par lequel ladite première section peut être reliée de telle sorte ou est reliée de telle sorte à ladite deuxième section que le manchon est maintenu, dans la position d'utilisation, sur le pied de l'utilisateur,
dans lequel, dans le cas d'espaces d'écoulement (4), en particulier d'espaces d'écoulement (4) critiques, dans lesquels un pli se forme dans l'espace d'écoulement (4) en particulier du fait de mouvements d'articulation et/ou de rotation du pied humain, le fluide peut s'écouler sur au moins un emplacement interrompu d'une mise en contact (6) linéaire entre deux espaces d'écoulement (4) pour garantir un écoulement ininterrompu du fluide.

2. Manchon selon la revendication 1, **caractérisé en ce que** la deuxième section est réalisée de manière asymétrique en comparaison avec la première section et en particulier en forme de trapèze avec des bords latéraux rectilignes et/ou à incurvation constante.

3. Manchon selon au moins l'une quelconque des revendications précédentes, **caractérisé en ce que** le dispositif de liaison présente au moins un élément de blocage, en particulier au moins un bouton-poussoir et/ou une fermeture auto-agrippante et/ou une boucle de liaison et/ou une bande élastique et/ou un cordon de soudure.

4. Manchon selon la revendication 3, **caractérisé en ce que** le dispositif de liaison présente plusieurs éléments de blocage, lesquels sont disposés en particulier de manière décalée les uns par rapport aux autres pour permettre une adaptation de dimensions variable au pied.

5. Manchon selon au moins l'une quelconque des revendications précédentes, **caractérisé en ce que** le dispositif de liaison est réalisé de telle manière qu'il entoure au moins en partie le manchon dans la position d'utilisation et exerce, dans ladite position dans laquelle il l'entoure, des forces auxiliaires sur le manchon, lesquelles contribuent au moins en partie à maintenir le manchon dans la position d'utilisation.

6. Manchon selon au moins l'une quelconque des revendications précédentes, **caractérisé en ce que** la pluralité de dispositifs de mise en contact (5) est disposée au moins en partie en forme de trame, de préférence sur les points de coin de rectangles imaginaires, par ailleurs de préférence sur les points de coin de carrés imaginaires.

7. Manchon selon au moins l'une quelconque des revendications précédentes, **caractérisé en ce que** l'au moins une première couche de matériau flexible et l'au moins une deuxième couche de matériau flexible entre au moins deux dispositifs de mise en contact (5), en particulier entre les points centraux des deux dispositifs de mise en contact (5), sont reliées de manière linéaire, en particulier de manière rectiligne.

8. Manchon selon l'une quelconque des revendications précédentes, **caractérisé en ce qu'**au moins un guidage parallèle du fluide est permis dans au moins deux espaces d'écoulement selon un angle prédéfini, en particulier de manière perpendiculaire ou parallèle, par rapport au pli pour garantir un écoulement ininterrompu du fluide.

9. Manchon selon l'une quelconque des revendications précédentes, **caractérisé en ce qu'**au moins un espace d'écoulement (4) varie dans la largeur, en particulier est agrandi pour garantir un écoulement ininterrompu du fluide.

10. Manchon selon au moins l'une quelconque des revendications précédentes, **caractérisé en ce que** le premier conduit d'arrivée (2) et le deuxième conduit d'arrivée (3) sont raccordés de manière sensiblement parallèle l'un par rapport à l'autre au corps de transfert de chaleur.

11. Système de traitement par la chaleur destiné à être utilisé lors du traitement par thermothérapie d'un pied humain, avec un manchon selon au moins l'une quelconque des revendications 1 à 10 et un dispositif d'alimentation en fluide, qui est mis au point pour être en communication fluidique avec le manchon et lequel peut être relié avec acheminement de fluide à l'au moins un premier conduit d'arrivée (2) et à l'au moins un deuxième conduit d'arrivée (3) du manchon et lequel est mis au point pour thermoréguler un fluide de transfert de chaleur sur une température adaptée à la thermothérapie, de manière préférée sur une température entre +5 °C et +35 °C, de manière davantage préférée entre +10 °C et +20 °C, de manière particulièrement préférée entre +14 °C et +16 °C et de manière très particulièrement préférée d'environ +15 °C, pour amener le fluide de transfert de chaleur ainsi thermorégulé au corps de transfert de chaleur (1) du manchon par l'intermédiaire du premier conduit d'arrivée (2) et pour évacuer le fluide de transfert de chaleur du corps de transfert de chaleur (1) par l'intermédiaire du deuxième conduit d'arrivée (3).

12. Utilisation d'un manchon selon l'une quelconque des revendications 1 à 10 et/ou d'un système de thermothérapie selon la revendication 11 pour le traitement cosmétique de la peau du pied humain, en particulier
- pour le traitement/la réduction de rides ou
- pour l'effet thermique sur le tracé des capillaires sur la peau ou
- pour la réduction non thérapeutique de tuméfactions cutanées.
